# EUROPÄISCHE PATENTANMELDUNG

(11) **EP 3 572 801 A1**
(43) Veröffentlichungstag der Anmeldung: **27.11.2019**
(21) Anmeldenummer: 19181326.0
(22) Anmeldetag: 25.08.2009
(51) Int. Cl.: G01N 21/85, G01N 21/11, B01L 3/00, F04B 43/08, G01N 35/10, G01N 1/40, F04B 43/12, G01N 21/05, G01N 21/64, G01N 21/78, G01N 21/03, G01N 21/84, G01N 33/18

(54) **PROZESS-ANALYSEGERÄT**

(62) Teilanmeldung aus: 09168536.2
(71) Anmelder: Hach Lange GmbH, 14163 Berlin (DE)
(72) Erfinder: LUNDGREEN, Ulrich, 33330 Gütersloh (DE); FARJAM, Aria, 40223 Düsseldorf (DE)
(74) Vertreter: Patentanwälte ter Smitten Eberlein-Van Hoof Rütten Partnerschaftsgesellschaft mbB

(57) **Zusammenfassung**

Die Erfindung bezieht sich auf ein Prozess-Analysegerät (10) mit einem Basismodul (14) und einem austauschbaren Kartuschenmodul (12).

Das Basismodul (14) weist einen Pumpenantrieb (16, 16') und einen Analyt-Sensor (20) ohne Fluidik-Messstrecke (60) auf. Das Kartuschenmodul (12) weist einen Flüssigkeits-Vorratstank (40,41), eine antriebslose Pumpmimik (36, 36'), die die Flüssigkeit aus dem Vorratstank (40,41) pumpt, und die Fluidik-Messstrecke (60) des Analyt-Sensors (20) auf.

Bei in das Basismodul (14) eingesetztem Kartuschenmodul (12) wird die Pumpmimik (36, 36') durch den Pumpenantrieb (16, 16') angetrieben und ist die Fluidik-Messstrecke (60) mit dem Analyt-Sensor (20) verbunden.

## Beschreibung

### Prozess-Analysegerät

Die Erfindung bezieht sich auf ein Prozess-Analysegerät, wie es beispielsweise in der Wasser-Analytik in Form einer Tauchsonde, einer Rohreinbausonde oder eines Labor-Analysegerätes eingesetzt wird.

Prozess-Analysegeräte führen quasi-kontinuierlich Analysen zur quantitativen Bestimmung eines Analyts in Wasser durch, und weisen hierfür einen oder mehrere Vorratstanks zur Bevorratung von Flüssigkeiten, z.B. Trägerflüssigkeiten und/oder Reagenzien auf, die für die Durchführung der Wasser-Analyse benötigt werden. Für den Transport der Flüssigkeiten in dem Analysegerät sind Pumpen und Flüssigkeitsleitungen vorgesehen.

Bei Erschöpfung eines Vorratstanks wird der betreffende Vorratstank wieder aufgefüllt. Im praktischen Betrieb treten ferner häufig Störungen auf, die insbesondere durch Ablagerungen und Auskristallisationen in den Flüssigkeitsleitungen oder auch in den flüssigkeitsführenden Teilen der Pumpen verursacht werden. Diese Störungen, sowie normale Abnutzung von Verschleißteilen wie z.B. Schlauchpumpen-Schläuchen, führen dazu, dass zusätzlich zu der regelmäßigen Auffüllung der Vorratstanks auch Wartungen und Reparaturen fällig werden, z.B. Austausch von Schläuchen.

Aufgabe der Erfindung ist es demgegenüber, ein Prozess-Analysegerät mit hoher Betriebssicherheit und geringem Wartungsaufwand zu schaffen.

Diese Aufgabe wird erfindungsgemäß durch ein Prozess-Analysegerät mit den Merkmalen des Patentanspruches 1 gelöst.

Das erfindungsgemäße Prozess-Analysegerät ist modular aufgebaut, und besteht aus einem ständig verwendeten Basismodul und einem austauschbaren Kartuschenmodul.

Das Basismodul weist einen Pumpen-Antriebsmotor und einen Analyt-Sensor ohne flüssigkeitsführende Messtrecke auf. Das Basismodul weist keinerlei Fluidik auf, also keine flüssigkeitsführenden Leitungen, Ventile, Messstrecken oder Pumpmimiken.

Das Kartuschenmodul dagegen weist mindestens einen Flüssigkeits-Vorratstank, eine antriebslose Pumpmimik zum Pumpen der Flüssigkeit aus dem Vorratstank, und eine Messstrecke für den Analysator auf. Die gesamte Fluidik ist also in dem Kartuschenmodul angeordnet. Unter Pumpmimik ist der fluidische Transportmechanismus zu verstehen, d.h. der Teil der Pumpe, der in direktem Kontakt zur Flüssigkeit steht und den Transport innerhalb des fluidischen Systems bewirkt.

Bei in das Basismodul eingesetztem Kartuschenmodul wird die Pumpmimik über das Kupplungsteil durch den Antrieb angetrieben, und ist die flüssigkeitsführende Messstrecke dem Analyt-Sensor derort zugeordnet, dass dieser Messungen von in der Flüssigkeit enthaltenen Analyten durchführen kann.

Bei Erschöpfung der Flüssigkeit in dem Vorratstank, beispielsweise einer Erschöpfung von Trägerflüssigkeit oder Reagenz, wird das Kartuschenmodul mit der gesamten Fluidik ausgetauscht. Der Austausch der gesamten Fluidik erfolgt also regelmäßig beziehungsweise verbrauchsgesteuert. Eine separate Wartung der Fluidik des Analysegerätes über das Auffüllen des Flüssigkeits-Vorratstanks hinaus entfällt. Der Wartungsaufwand ist dadurch erheblich reduziert. Durch das regelmäßige beziehungsweise verbrauchsgesteuerte Austauschen der gesamten Fluidik werden Störungen, die durch Ablagerungen in der Fluidik oder Verschleiß fluidischer Komponenten, z.B. Pumpenschläuche, verursacht werden können, auf ein Minimum reduziert.

Vorzugsweise ist die Pumpmimik als Peristaltik ausgebildet, und besonders bevorzugt als pneumatische peristaltische Membranpumpe. Eine peristaltische Pumpmimik ist konstruktiv sehr einfach, und kann beispielsweise von einem flexiblen Abschnitt der Flüssigkeitsleitung gebildet werden, der von dem Kopplungsteil in einer Längsrichtung fortlaufend verformt wird.

Das Kopplungsteil kann beispielsweise in Form von drei in Längsrichtung hintereinander angeordneten Quetschkolben ausgebildet sein, die in einer Längsrichtung nacheinander den Flüssigkeitsleitungs-Abschnitt flüssigkeitsdicht abquetschen, und auf diese Weise eine peristaltische Bewegung erzeugen. Eine besonders bevorzugte Ausführungsform ist die pneumatische Peristaltikpumpe, bei der die mechanische Kopplung durch Anlegen von Über- bzw. Unterdruck auf eine mehrstufige Membranpumpe erzeugt wird. Der Pumpenantrieb kann beispielsweise elektromechanisch oder pneumatisch ausgebildet sein.

Ein Lufteintrag in einer Flüssigkeitsleitung stellt für eine peristaltische Pumpmimik kein Problem dar, da diese gegen Luft ansaugen kann. Ferner kann bei Verwendung einer peristaltischen Pumpmimik auf Ventile, insbesondere auf anfällige RückschlagVentile verzichtet werden. Die peristaltische Pumpmimik bietet ferner die Möglichkeit, mit einer einzigen peristaltischen Pumpmimik mehrere Leitungen synchron, das heißt, mehrere Flüssigkeiten gleichzeitig, pumpen zu können. Schließlich ist die peristaltische Pumpmimik eine ausgereifte, preiswerte und äußerst zuverlässige Pumptechnik.

Gemäß einer bevorzugten Ausgestaltung weist das Basismodul mehrere voneinander unabhängige Pumpenantriebe auf, die mehreren voneinander unabhängigen Pumpmimiken in dem Kartuschenmodul zugeordnet sind. Zwar können grundsätzlich auch mit einem einzigen Pumpenantrieb mehrere Pumpmimiken angetrieben werden. Vorteilhaft ist jedoch, jeder Pumpmimik in dem Kartuschenmodul basismodulseitig einen eigenen Pumpenantrieb zuzuordnen. Auf diese Weise können mehrere Flüssigkeitsströme unabhängig voneinander gesteuert werden.

Vorzugsweise weist das Kartuschenmodul mehrere Vorratstanks auf. Als Vorratstank ist vorliegend ein Tank für ein Reagenz, eine Trägerflüssigkeit, eine Reinigungsflüssigkeit oder auch für Abfallflüssigkeit zu verstehen.

Gemäß einer bevorzugten Ausgestaltung weist der Analyt-Sensor basismodulseitig ein Fotometer zur fotometrischen Bestimmung eines Analyts in einer Wasserprobe auf. Die Wasserprobe kann beispielsweise dialytisch beispielsweise aus Abwasser gewonnen werden. Hierzu weist das Kartuschenmodul in einer bevorzugten Ausführungsform eine Dialysemembran auf. Besonders bevorzugt ist das vorliegende Analysegerät als flüssigkeitsdichte Abwasser-Tauchsonde ausgebildet.

Im Folgenden wird die Erfindung anhand der Zeichnungen näher erläutert.

Es zeigen:
- Figur 1: eine schematische Darstellung eines als Tauchsonde ausgebildeten Analysegerätes,
- Figur 2: eine erste Ausführungsform eines basisseitigen pneumatischen Pumpenantriebs und einer damit zusammenwirkenden kartuschenseitigen Pumpmimik, und
- Figur 3: eine zweite Ausführungsform eines basisseitigen elektromechanischen Pumpenantriebs und einer damit zusammenwirkenden kartuschenseitigen Pumpmimik.

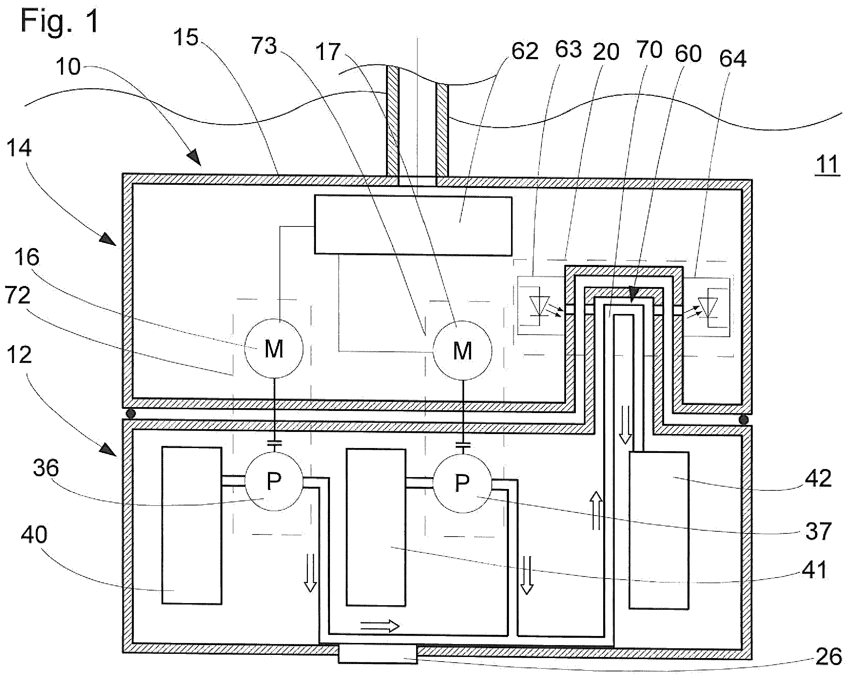

In der Figur 1 ist schematisch ein Prozess-Analysegerät 10 dargestellt, das als Tauchsonde ausgebildet ist, und der Analyse von Wasser 11 zur Regelung eines Klärprozesses dient. Das Analysegerät 10 ist zweiteilig und modular aufgebaut, und besteht aus einem flüssigkeitsdichten Basismodul 14 und einem austauschbaren flüssigkeitsdichten Kartuschenmodul 12. Das Basismodul 14 weist ein Gehäuse 15 auf, in das bzw. an das das Kartuschenmodul 12 ein- bzw. angesteckt ist.

Das Basismodul 14 weist einen als Fotometer ausgebildeten Analyt-Sensor 20 ohne Fluidik-Messtrecke 60 auf, die in dem Kartuschenmodul 12 vorgesehen ist. Der Analyt-Sensor 20 weist basismodulseitig eine Lichtquelle 63 und einen Detektor 64 in Form einer Photozelle auf.

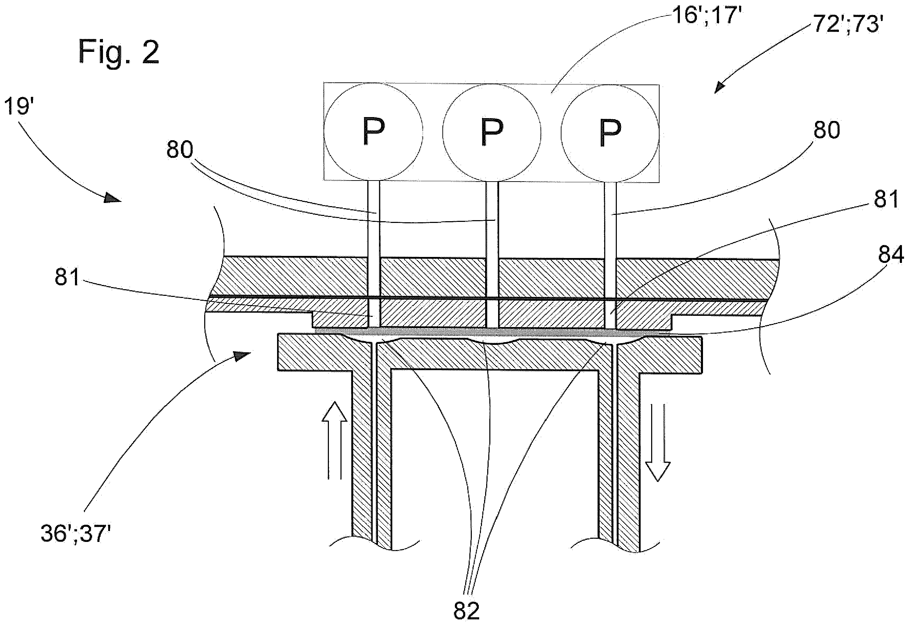

Wie in Figur 2 dargestellt ist, kann die Pumpe gemäß einer ersten Ausführungsform pneumatisch ausgebildet sein, wobei der Pumpenantrieb 16' als Luftpumpe ausgebildet ist, die drei Luftkanäle 80 sukzessive mit Überdruck und ggfs. auch mit Unterdruck beaufschlagt, die mit entsprechenden Luftkanälen 81 des Kartuschenmoduls 12 gekoppelt sind. Die kartuschenmodulseitigen Luftkanäle 81 sind durch eine Pumpmembran 84 verschlossen, unter der drei Pumpkammern 82 gebildet sind.

Wird in dem Luftkanal 80 durch den Pumpenantrieb 16' Überdruck angelegt, dehnt sich die Pumpmembran 84 in die darunter liegende Pumpkammer 82 aus und verdrängt die darin befindliche Flüssigkeit. Wird Unterdruck angelegt, legt sich die Pumpmembran 84 glatt an die Oberfläche der Luftseite an und die Pumpkammer 82 füllt sich wieder mit Flüssigkeit. Durch wechselndes Anlegen von Über- und Unterdruck in der entsprechenden Reihenfolge der drei aufeinanderfolgenden Pumpkammern 82 wird eine peristalitsche Fortbewegung der Flüssigkeit durch alle drei Pumpkammern 82 und im nachfolgenden fluidischen System erreicht

In dem Pumpenantrieb 16' sind drei nicht dargestellte Ventile vorgesehen, die entweder Überdruck oder Unterdruck in den jeweiligen Luftkanal 80 durchschalten.

Die in den Figuren nicht dargestellten Ventile sind demnach die Kopplung zwischen der Druckluftpumpe und der Pumpmimik 36'.

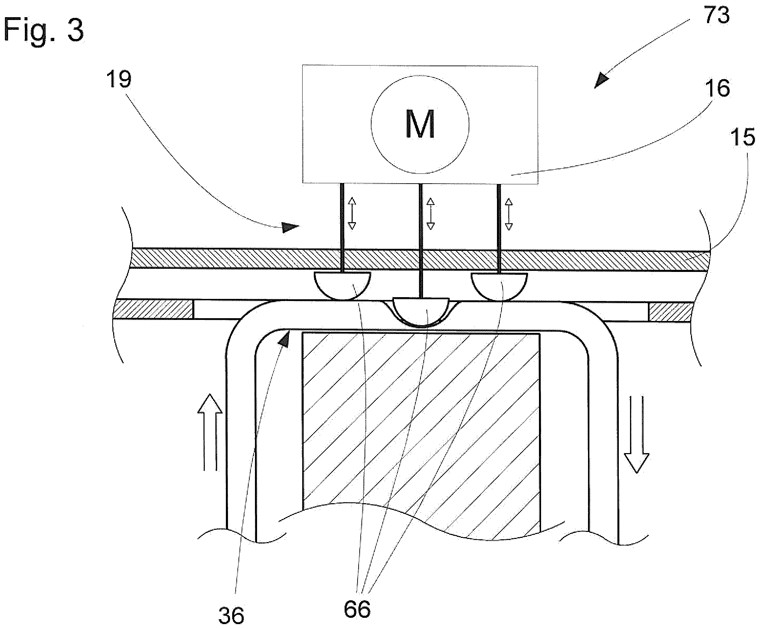

Das Basismodul 14 weist als Pumpenantrieb alternativ einen Antriebsmotor 16,17 mit einem Kopplungsteil 19 auf, das in der Figur 3 dargestellt ist. Das Kopplungsteil 19 besteht aus drei peristaltischen Quetschkolben 66, die eine peristaltische Bewegung in einer als flexibler Leitungsabschnitt ausgebildeten Pumpmimik 36 erzeugen können.

Zur Steuerung des Analysegerätes 10 weist das Basismodul 14 ferner eine Steuerung 62 auf.

Das Kartuschenmodul 12 weist drei Vorratstanks 40,41,42 auf, in denen eine Trägerflüssigkeit, ein Reagenz sowie Abfallflüssigkeit gelagert sind. Der Trägerflüssigkeits-Tank 40 und der Reagenz-Tank 41 sind jeweils über eine Flüssigkeitsleitung an eine eigene peristaltische Pumpmimik 36,37 angeschlossen, durch die die Trägerflüssigkeit beziehungsweise das Reagenz fein dosiert gepumpt werden kann. Der dritte Vorratstank 42 ist ein Tank für Abfallflüssigkeit, in den die von dem Analysator 20 kommende Abfall-Flüssigkeit fließt.

Die peristaltischen Pumpmimiken 36,37 der zweiten Ausführungsform sind zusammen mit den zugeordneten Kopplungsteilen 19 derart ausgelegt, dass ein unerwünschter Rückfluss oder Leckfluss von Trägerflüssigkeit oder Reagenz nicht auftreten kann. Die peristaltischen Pumpmimiken 36,37 sind kartuschenmodulseitig im Wesentlichen durch flexible Abschnitte der betreffenden Flüssigkeitsleitung gebildet, die durch die betreffenden Kopplungsteile 19 der zugeordneten Antriebsmotoren 16,17 angetrieben werden. Die Fluidik- Pumpe 72,73 der zweiten Ausführungsform wird also von dem Antriebsmotor 16, dem Kopplungsteil 19 und der Pumpmimik 36 gebildet.

Ferner weist das Kartuschenmodul 12 eine ionenselektive Dialyse-Membran 26 auf, durch die Analyt-Moleküle aus dem Wasser 11 in die innenseitig vorbei fließende Trägerflüssigkeit wandern können, Partikel, Makromoleküle und Mikroorganismen aber von Durchtritt abgehalten werden. Die Membran weist bevorzugt eine Porengröße von weniger als 2 µm auf.

Schließlich weist das Kartuschenmodul 12 die Fluidik-Messtrecke 60 auf, die von einem geraden Leitungsabschnitt 70 gebildet wird, dessen Längsenden transparente, lichtdurchlässige Fenster für die Fotometrie im Analyt-Sensor 20 aufweisen.

Wenn das Kartuschenmodul 12 an das Basismodul 14 montiert ist, ist die Messtrecke 60 genau ausgerichtet mit den basismodulseitigen Teilen des Analyt-Sensors 20. Ferner sind die Kopplungsteile 19 der Pumpen 72, 73 in peristaltischem Eingriff mit den kartuschenmodulseitigen Pumpmimiken 36.

Im Analysebetrieb wird aus dem Trägerflüssigkeits-Vorratstank 40 durch die zugeordnete Pumpe 72 Trägerflüssigkeit zu der Dialysemembran 26 gepumpt, durch die Analyt-Ionen aus dem Wasser 11 in die Trägerflüssigkeit wandern. Anschließend wird der mit den Analyt-Ionen angereicherten Trägerflüssigkeit ein Reagenz aus dem Reagenz-Vorratstank 41 durch die zugeordnete Pumpe 73 beigefügt, durch das der zu untersuchende Analyt in der Trägerflüssigkeit entsprechend seiner Konzentration eingefärbt wird. Die eingefärbte Probe wird zu der Messstrecke 70 gepumpt und dort durch den Analyt-Sensor 20 fotometrisch untersucht. Anschließend wird die Probe in den Abfall-Vorratstank 42 gepumpt.

Bei Erschöpfung von Reagenz oder Trägerflüssigkeit in den entsprechenden Vorratstanks 40,41, bei drohender Überfüllung des Abfall-Vorratstanks 42 oder bei einer Fehlfunktion, die möglicherweise auf eine der Komponenten des Kartuschenmoduls 12 zurückgeht, wird das Kartuschenmodul 12 ausgetauscht. Hierbei wird eine nicht dargestellte Verriegelung entriegelt und das Kartuschenmodul 12 axial nach unten von dem Basismodul-Gehäuse 15 abgezogen. Hierbei wird das Pumpen-Kopplungsteil 19 von der Pumpmimik 36 gelöst und wird die Messtrecke 60 aus dem Analyt-Sensor 20 entfernt. Anschließend kann ein neues Kartuschenmodul 12 an das Gehäuse 15 angesetzt werden, wobei die Pumpen-Kopplungsteile 19 wieder mit den Pumpmimiken 36 verbunden werden und die Messstrecke 60 in den Analysator 20 eingeführt wird.

## Patentansprüche

1. Prozess-Analysegerät (10) mit einem Basismodul (14) und einem austauschbaren Kartuschenmodul (12), wobei
das Basismodul (14) einen Pumpenantrieb (16, 16') und einen Analyt- Sensor (20) ohne Fluidik-Messstrecke (60) aufweist, und
das Kartuschenmodul (12) einen Flüssigkeits-Vorratstank (40,41), eine antriebslose Pumpmimik (36,36'), die die Flüssigkeit aus dem Vorratstank (40,41) pumpt, und die Fluidik-Messstrecke (60) des Analyt-Sensors (20) aufweist,
wobei bei in das Basismodul (14) eingesetztem Kartuschenmodul (12) die Pumpmimik (36,36') mit dem Antrieb (16,16') verbunden und durch den Antrieb (16,16') angetrieben wird, und die Fluidik-Messstrecke (60) mit dem Analysator (20) verbunden ist.

2. Prozess-Analysegerät (10) nach Anspruch 1, wobei die Pumpmimik (36, 36') als Peristaltik ausgebildet ist.

3. Prozess-Analysegerät (10) nach Anspruch 1 oder 2, wobei die Pumpmimik (36') als peristaltische Membranpumpe ausgebildet ist, und besonders bevorzugt pneumatisch angetrieben wird.

4. Prozess-Analysegerät (10) nach einem der vorangegangenen Ansprüche, wobei das Kartuschenmodul (12) mehrere Vorratstanks (40,41) aufweist.

5. Prozess-Analysegerät (10) nach einem der vorangegangenen Ansprüche, wobei das Basismodul (14) mehrere voneinander unabhängige Antriebe (16,17,16',17') aufweist, die mehreren voneinander unabhängigen Pumpmimiken (36,37,36',37') in dem Kartuschenmodul (12) zugeordnet sind.

6. Prozess-Analysegerät (10) nach einem der vorangegangenen Ansprüche, wobei der Analyt-Sensor (20) ein optischer Detektor ist, der die Bestimmung eines Analyts in der Fluidik-Messstrecke (60) erlaubt.

7. Prozess-Analysegerät nach Anspruch 6, wobei der optische Detektor die Absorption, die Fluoreszenz oder die Lumineszens einer Wasserprobe ermittelt.

8. Prozess-Analysengerät nach einem der vorangegangenen Ansprüche 1 bis 5, wobei der Analyt-Sensor (20) eine nicht-optische Detektionseinheit ist, und als elektrochemischer Sensor, Leitfähigkeitssensor für Strom oder Wärme oder als Elektrophoresesystem ausgebildet ist.

9. Prozess-Analysegerät (10) nach einem der vorangegangenen Ansprüche, wobei das Analysegerät (10) als Abwasser-Tauchsonde ausgebildet ist.

10. Prozess-Analysegerät (10) nach einem der vorangegangenen Ansprüche, wobei das Kartuschenmodul (12) eine Dialyse-Membran (26) aufweist.

11. Prozess-Analysegerät (10) nach einem der vorangegangenen Ansprüche, wobei das Kartuschenmodul (12) flüssigkeitsdicht gekapselt ist, so dass die darin enthaltenen Flüssigkeiten nicht nach Außen treten können.
